# EUROPEAN PATENT APPLICATION

(11) **EP 2 630 945 A1**
(43) Date of publication of application: **28.08.2013**
(21) Application number: 13155278.8
(22) Date of filing: 14.02.2013
(51) Int. Cl.: A61K 8/30, A61Q 19/10, A61K 8/02

(54) **Combination of a vegetable sponge obtained from the dried fruit of a plant of the family Cucurbitaceae of the genus "Luffa" with surfactant and / or cosmetic substances**

(30) Priority: 23.02.2012 IT GE20120013 U
(71) Applicant: Locatelli, Sergio, 15067 Novi Ligure (IT)
(72) Inventor: Locatelli, Sergio, 15067 Novi Ligure (IT)
(74) Representative: Porsia, Attilio

(57) **Abstract**

Combination of a vegetable sponge (4) obtained by drying the fruit of a plant of the family of the Cucurbitaceae of the genus "Luffa" provided at its interior with a plurality of cylindrical cavities (3) with a surfactant product, by the introduction of said surfactant product into the cavities (3) of the sponge (4). Said surfactant product is a solid, semisolid or liquid soap, which is shaped in form of rods (5) which are introduced into the cavities (3) of the sponge (4).

## Description

The present invention relates to the combination of a vegetable sponge obtained from a plant of the family Cucurbitaceae of the genus "Luffa" with solid or semisolid surfactant products, such as soaps, detergents, or the like.

It is well known that the fruit of some Cucurbitaceae of the genus "Luffa", and in particular that of the species *Luffa cylindrica*, if macerated and completelydried after complete maturation, is transformed into a lattice of hollow fibers commercialized under the name of "vegetable sponge".

These fruits contain at their inside cylindrical or substantially cylindrical cavities containing the seeds of the plant which, after complete drying and ripening of the fruit, are expelled at the outside, leaving these cavities free.

Surprisingly, it has been discovered that these cavities of the sponge are apt to accommodate a hydro-soluble solid or semi-solid surfactant product such as for example a solid or semi-solid soap, releasing it in a gradual manner only when the sponge is wetted and used.

Furthermore, this surfactant product contained at the inside of the sponge remains dry between a use and the following one thanks to the fact that the conformation of the sponge allows the drainage of excess water, maintaining the organoleptic integrity of the product.

Further objects and advantages of the combination of a vegetable sponge with a detergent product according to the invention will be better apparent in the course of the following description thereof, made with reference to the accompanying drawing, in which:
Figure 1 is an elevational view of a vegetable sponge or "luffa";
Figure 2 is a longitudinal sectional view of the sponge of Figure 1, showing the internal cylindrical cavities, housing the detergent product, and
Figure 3 is a cross-sectional view of the sponge of Figures 1 and 2.

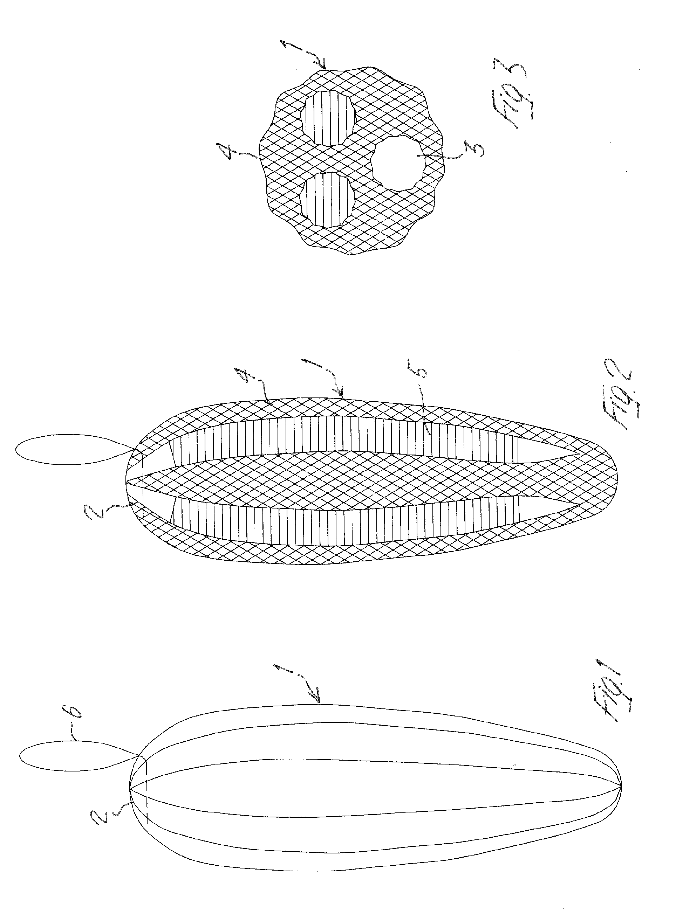

With reference to the drawings, and with particular reference to Figure 1 of same, with numeral 1 the fruit of luffa cylindrical is shown. Such fruit has on one side a operculum 2 which, when the fruit is ripe, it detaches putting in communication with the external environment a series of three or four cylindrical cavities 3 containing the seeds. When the fruit of the luffa is macerated, exfoliated and dried, it is transformed into a lattice of hollow fibers 4 known as "vegetable sponge". The sponge 4 of luffa has an excellent ability to gently exfoliate the skin, removing dead cells and reactivating the blood microcirculation.

In a surprisingly manner it has been found that it is possible to increase and implement such action, by introducing inside the cavities 3 of the sponge 4 a solid or semi-solid detergent.

Advantageously, the solid or semi-solid detergents to be introduced into the cavity 3 of the sponge 4 are shaped in the form of cylindrical rods 5 having dimensions from 13 to 15 mm of diameter and length from 130 to 150 millimeters. Once that said rods 5 are inserted in the cavities 3 of the sponge, the fibrous matrix and the conical shape of these cavities does not allow them to escape.

The introduction of these rods 5 of solid or semi-solid deterget in the cavities 3 of the sponge 4 allows a more practical use and a more efficient consumption of both the sponge 4 and of the detergent product 5 contained in the same, with numerous advantages for the user:
- The rods 5 of detergent are consumed very slowly, with the release of foam on the skin of the user only when the sponge 4 is used;
- There is no risk that the dispensed detergent be over-or under dosed, moreover the rods 5 of detergent remain dry inside of the sponge between one use and the following thanks to the fact that the conformation of the sponge allows the drainage of excess water, maintaining the organoleptic integrity of the product;
- Only one product is sufficient for the complete cleansing.

As examples of compounds which can be introduced into the cavity 5 are cited:
- Solid neutral soap
- Liquid Soap

In conjunction with the detergent surfactants products, it is possible to introduce into the cavities 3 of the sponge also cosmetic products and / or emollients such as glycerin, or moisturizing creams, or mixtures of these ingredients.

Advantageously, around the end from which the operculum 2 was detached, a string 6 is passed, which in addition to closing the inlet of the ducts 3, also provides an element for hanging the sponge when the same is not used.

Obviously the present invention is not limited to what is described and illustrated, but includes all those variations and modifications made to a vegetable sponge combined with a cleaning and / or cosmetic substance, apt to achieve the same utility, substantially as claimed in the appended claims.

## Claims

1. Combination of a vegetable sponge (4) obtained by drying the fruit of a plant of the family of the Cucurbitaceae of the genus "Luffa" provided at its interior with a plurality of cylindrical cavities (3) with a surfactant product, **characterized in that** said surfactant product is introduced into said cavities (3) of the sponge (4).

2. Sponge according to claim 1, wherein said surfactant product is a solid, semisolid or liquid soap.

3. Sponge according to claims 1 or 2, wherein in said cavities (3) are introduced, together with said surfactant product, emollients, such as glycerin, or moisturizing creams, or mixtures of these ingredients.

4. Sponge according to any one of the preceding claims wherein said solid or semisolid surfactant products are shaped in the form of rods (5) which are introduced into the cavities (3) of the sponge (4).

5. Sponge according to any one of the preceding claims, wherein around the end of the sponge (4) communicating with the internal cavities (3) of the same, a string element (6) is passed which is apt to close the outlets of the internal cavities (3) of the sponge (4).

6. Sponge according to any one of the preceding claims, wherein the ends of said string (6) are used as hanger for hanging the sponge.
